# EUROPEAN PATENT SPECIFICATION

(11) **EP 0 812 191 B1**
(45) Date of publication and mention of the grant of the patent: **20.07.2005**
(21) Application number: 95916363.5
(22) Date of filing: 11.04.1995
(51) Int. Cl.: A61K 31/41, A61K 31/70, A61K 38/10, A61K 39/395, C07H 5/06, C07H 21/02, C07H 21/04, C07K 7/08, C07K 16/18, C07K 16/32, G01N 33/48, A61P 35/00

(54) **PEPTIDES FOR THE TREATMENT OF CANCER**
PEPTIDE ZUR BEHANDLUNG VON KREBS
PEPTIDES POUR LE TRAITEMENT DU CANCER

(43) Date of publication of application: 17.12.1997
(73) Proprietor: Bogoch, Samuel, Dr., New York, New York 10028 (US); Bogoch, Elenore, S., New York, New York 10028 (US)
(72) Inventor: Bogoch, Samuel, Dr., New York, New York 10028 (US); Bogoch, Elenore, S., New York, New York 10028 (US)
(74) Representative: Mallalieu, Catherine Louise
(86) International application number: PCT/US1995/004553
(87) International publication number: WO 1996/032106

(56) References cited:
- WO-A-93/08808
- US-A- 4 840 915
- US-A- 4 870 061
- US-A- 4 918 170
- US-A- 5 220 008
- S. BOGOCH ET AL.: "AGLYCO PATHOLOGY OF VIRAL RECEPTORS IN DEMENTIAS" ANNALS OF THE NEW YORK ACADEMY OF SCIENCES; DIVERSITY OF INTERACTING RECEPTORS (CONFERENCE WASHINGTON, D.C., USA; MAY 25-28, 1994), vol. 757, 1995, pages 413-417, XP008003395 NEW YORK, N.Y., US
- DATABASE BIOSIS [Online] BIOSCIENCES INFORMATION SERVICE, PHILADELPHIA, PA, US; 1995 BOGOCH S ET AL: "Aglyco pathology of viral receptors in dementias." Database accession no. PREV199598533341 XP002199626 & ANNALS OF THE NEW YORK ACADEMY OF SCIENCES, vol. 757, 1995, pages 413-417, Conference;Washington, D.C., USA; May 25-28, 1994, receptors. 1995 New York Academy of Sciences 2 East 63rd Street, New York, New York 10021, USA ISSN: 0-89766-924-X
- DATABASE MEDLINE [Online] August 1979 (1979-08) KORNBLITH P L ET AL: "Growth-inhibitory effect of diphenylhydantoin on murine astrocytomas." Database accession no. NLM481731 XP002199627 & NEUROSURGERY. UNITED STATES AUG 1979, vol. 5, no. 2, August 1979 (1979-08), pages 259-263, ISSN: 0148-396X
- S. BOGOCH ET AL.: "MALIGNIN ANTIBODY AND EARLY MALIGNANCY." THE LANCET , vol. 337, 20 April 1991 (1991-04-20), page 977 XP002199625 LONDON, GB
- CANCER DETECTION AND PREVENTION, Volume 17, Number 1, issued 1993, S. BOGOCH et al., "Malignin Antibody Returns to Normal on Successful Treatment of breast Cancer", page 180, Abstract 276/35.
- JOURNAL OF THE NATIONAL CANCER INSTITUTE, Volume 71, Number 2, issued August 1983, S. HAKOMORI et al., "Glycosphingolipids as Tumor-Associated and Differentiation Markers", pages 231-251.
- THE MERCK INDEX, 8th Edition, issued 1968, ENTRY ENTITLED, "Diphenylhydantoin", page 388.
- SIGMA CHEMICAL COMPANY (Catalogue), issued 1993, "Biochemical Compounds for Research and Diagnostic Reagents", page 459, Product No. G-4875.
- AMERICAN JOURNAL OF PSYCHIATRY, Volume 123, Number 8, issued February 1967, R.J. CAMPBELL et al., "Cerebrospinal Fluid Glycoproteins in Schizophrenia", pages 952-962.

## Description

This invention concerns the discovery of products and methods to aid in the diagnosis and treatment of disorders of conjugated carbohydrate constituents of living organisms which contribute to cell dysfunction and cell death.

The present invention teaches that where normal carbohydrate constituents are covalently bound or otherwise closely associated with other cell structures in the form of glycoconjugates, these carbohydrate constituents can contribute to cell stability, to receptor and recognition functions of the cell, and to the protection of cell constituents from damage by both native and foreign substances. When these normal carbohydrate constituents are reduced in concentration or otherwise structurally altered, together defined here as aglyco states, the stability, receptor, recognition, and protective functions of these carbohydrates are diminished or lost, and cell dysfunction and cell death result, with resultant disease states depending on the location of the cell dysfunction or cell death. Evidence here presented indicates that these disease states , in the nervous system for example, as a result of cell dysfunction or cell death (i.e. cell loss), include but are not limited to brain tumors.

This invention teaches that these disorders can be diagnosed by the products of aglyco pathology as follows: 1) by direct determination of structural changes in the nervous system glycoconjugates which changes produce novel aglyco products; or 2) because these novel aglyco products may act as antigens, by determination of the novel antibody products produced by the body against the novel aglyco antigenic products. Thus these aglyco cell antigen products may be sufficiently different from the normal constituents that the body recognizes the new aglyco products as foreign and makes antibodies against them, which antibodies can have a deleterious or desirable effect on the cell and cause cell death (eg. in normal developing brain, not desirable; in brain tumors, desirable). The identification of these aglyco products by direct determination or by determination of the antibodies raised against them, can be an aid to, or the basis for, diagnosis of the disorder as well as the basis for its treatment.

Many seemingly unrelated observations in the past, which were not understood, and could not be understood in terms of cell dysfunction, cell death, and specific disease states, can now be understood in the light of the present invention which defines for the first time the state of, and the consequences of, aglyco pathology, its products, as well as products and processes for its detection and treatment.

According to one aspect of the present invention there is provided a peptide where the sequence of the amino acids is tryosine-lysine-alanine-glycine-valine-alanine-phenylalanine-leucine-histidine-lysine-lysine-asparagine-aspartic acid-isoleucine-asparagine-glutamic acid or derivative thereof.

According to another aspect of the present invention there is provided a peptide where the sequence of the amino acids is glycine-leucine-serine-aspartic acid-glycine-serine-asparagine-theronine-glutamic acid-serine-aspartic acid-isoleucine or derivative thereof.

According to another aspect of the present invention there is provided use of a peptide of the present invention in the preparation of a medicament for the treatment of cancer.

According to yet another aspect of the present invention there is provided an ex vivo process for diagnosing cancer or susceptibility to cancer in a subject related to expression or activity of a polypeptide of the present invention in a subject comprising analysing for the presence or amount of said polypeptide or an antibody to said polypeptide, in a sample derived from said subject.

According to yet a further aspect of the present invention there is provided a vaccine composition comprising a polypeptide as defined in the present invention and a pharmaceutically effective carrier.

The significance of the cell damage or cell death which may be a consequence of influenza virus infection in the developing brain is seen in this Example which relates influenza virus infection to neuronal cell injury, cell death and thus loss, and to schizophrenia, and demonstrates directly and quantitatively the nature of the aglyco products involved.

Earlier quantitative neurochemical studies suggested that neuraminic acid and hexosamine are part of a glycoconjugate intercellular recognition 'sign-post' system which forms the neural networks underlying normal brain development and behavior. Recent histological studies of schizophrenic brain reveal neuronal disorganization which might result from some developmental injury. Epidemiological studies now suggest that infection prenatally with influenza virus , which contains neuraminidase, predisposes to schizophrenia. That conjugated neuraminic acid and hexosamine in cerebrospinal fluid are quantitatively decreased in schizzophrenia is shown here in a double-blind study.

The frequency of schizophrenia appears to be increased, from recent epidemiological observations, in individuals whose mothers had influenza virus infection during the second trimester of the individual's gestation (1-9), and in populations with a high frequency of ganglioside and other glycolipid disorders (10). Histological studies demonstrate neuronal cell loss and neuronal disorganization in schizophrenic brain (11-15). The removal of neuraminic acid from its conjugates by the influenza virus enzyme neuraminidase (sialidase) might be responsible for injury to neuronal cell recognition molecules in the immature brain. The above recent findings in epidemiology and histology are relevant to quantitative neurochemical data which demonstrates a decrease in the actual concentration of neuraminic acid and hexosamine conjugates in cerebrospinal fluid (CSF) of schizophrenic patients (16-23,36,37,39), here confirmed, and the related work on glycoconjugate (glycolipid, glycoprotein) recognition substances in brain (24-33) which led to the 'sign-post' hypothesis of neuronal connectivity and nervous system maturation (34-35).

Work in this laboratory led to the conclusion that glycoconjugates in nervous system are recognition substances (24-35). After the structure of the brain gangliosides was first sufficiently determined, gangliosides *in vitro* were shown to act both as receptors for influenza virus, and as effectors, in stimulating clam heart and smooth muscle. *In vivo,* additional ganglioside administered intracerebrally acted as a "decoy" and inhibited infection of brain neurones by influenza virus. Human brain glycoproteins were isolated in bulk, separated, and partially characterized in terms of their carbohydrate constituents. Glycoconjugates were then shown to be present in synaptic membranes, to be involved in mammalian neural development, and in training and learning tasks in pigeons. The 'sign-post' function of glycoconjugates in nervous system was postulated with reference to the establishment of neural networks, both during developmental maturation and with experience. Schizophrenic patients were shown to have a significant decrease of conjugated neuraminic acid and hexosamine in CSF compared to other psychotic disorders and normals (16-23). This decrease was quantitatively proportional to the severity of the illness, and increased toward normal with improvement in clinical status (36,37,39).

We here illustrate a double-blind study which demonstrates the decrease in conjugated neuraminic acid and hexosamine in CSF of schizophrenic as compared to non-schizophrenic psychotic patients. Of nineteen patients two patients were excluded because the clinical diagnoses were not known. The remaining 17 were designated "schizophrenic" (N=11) when this diagnostic term was used, and "non-schizophrenic" other psychoses (N=6) when schizophrenia was not the diagnosis. These diagnoses were made by careful clinical work-up including full history and assesment of mental status with the Psychotic Characteristics Scale (39) by research psychiatrists who had completed a minimum of three years of psychiatric residency training, and the diagnoses were confirmed by senior staff psychiatrists.

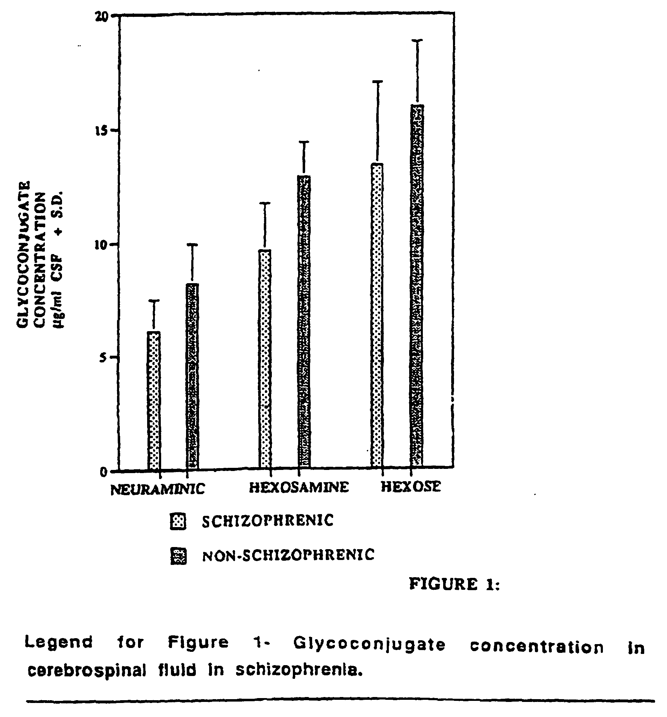

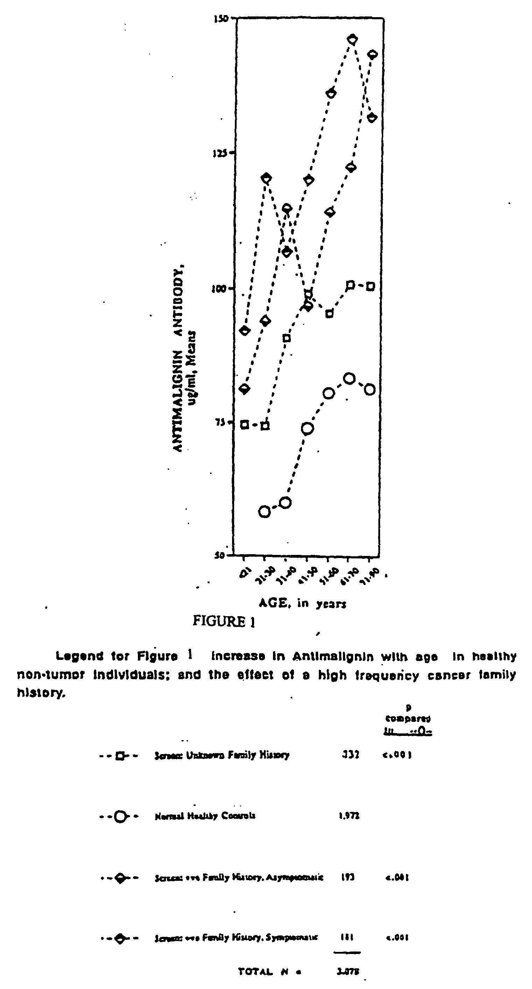

Lumbar CSF specimens, 5 to 18 ml., coded, and without clinical information, were shipped in dry ice to the laboratory. Each of the specimens was lyophilized, dialyzed against distilled water exhaustively (cellophane pore size approx. MW 12,000 Daltons) at 0 - 5°C to remove free neuraminic acid and free hexose, and the non-dialyzable fraction was quantitatively analyzed in duplicate for conjugated neuraminic acid by the Bial's orcinol and thiobarbituric acid (TBA) methods, conjugated hexosamine, and conjugated hexose, all as previously described (19). After the neurochemical tests for all specimens were completed, the code was broken. Despite the small sample size, conjugated neuraminic acid (p<.025), and conjugated hexosamine (p<.006), but not conjugated hexose, were statistically significantly lower in concentration in the schizophrenic group by two-tailed t test. Values of conjugated neuraminic acid in 81.8% of schizophrenics were less than 7.5 µg/ml CSF, and in 83.3% of non-schizophrenics were greater than 7.5 µg/ml CSF (see Figure 1). In addition, in three patients, one non-schizophrenic and two schizophrenic patients, who had repeated CSF drawn weeks apart, reproducibility was obtained in the neurochemical analysis of the randomized blind specimens. Determination of conjugated neuraminic acid by the TBA method was shown not to be reliable (38,39). It should be emphasized that in the present and all previous studies, both in vitro and in vivo , it is only the conjugated and never the free neuraminic acid which is found to be biologically active or decreased in schizophrenia.

Thus, previous neurochemical data led to the normal 'sign-post' hypothesis, but the nature fof the pathology of glycoconjugates was not understood. That is, the meaning of a decrease in the concentration of neuraminic acid and hexosamine, as glycoconjugates, was not understood. That a decrease in these glycoconjugates pre- or post-natally, due to virus or other acute or chronic cause, is critical in the pathogenesis of schizophrenia was not realized. Consideration of the epidemiological evidence of the relation of influenza virus infection in the second trimester of pregnancy to frequency of schizophrenia resulting , and histological evidence of brain cell loss and disorganization, taken together with direct evidence of the quantitative decrease in neuraminic acid and hexosamine concentration in glycoconjugates, has led to the present discovery of aglyco pathology.

EXAMPLE 4. Treatments for schizophrenia which are beneficial increase the concentration of glycoconjugates in the nervous system concomitant with patient improvement. Reference 39 describes the increase (+) or decrease (-) in absolute amounts of glycoprotein carbohydrate constituents in the cerebrospinal fluid in 65 instances of improvement, "no change", or worsening in the clinical status of 47 schizophrenic patients during entry into and recovery from a psychotic state treated with electroshock or drugs.

EXAMPLE 5. Administration of a neuraminic acid and hexosamine precursor to schizophrenic patients results in improvement. D-glucosamine HCl, 200 mg per day, was administered per os to chronic hospitalized schizophrenic patients for 30 days. The Psychotic Characteristics rating scale was used, together with overall clinical

### PREPARATIVE EXAMPLE

Increase in brain glycoconjugate hexose and in the incorporation of 14C glucose in pigeons during training.

The brains of pigeons at rest compared to training in a Skinner box were compared for a) the absolute amount of glycoconjugate hexose and b) the incorporation of ¹⁴C-glucose into brain glycoconjugates.

The results are as follows:

### a) ABSOLUTE CONCENTRATION OF GLYCOCONJUGATE HEXOSE

| | Number of Pigeons | Glycoconjugate Hexose mean mg/g wet weight brain |
|---|---|---|
| Resting, never trained | 21 | 0.63 |
| Resting, 3 to 11 months | | |
| post-training | 11 | 0.69 |
| Training: | | |
| 10 minutes | 1 | 9.0 |
| 20 minutes | 1 | 8.8 |
| 30 minutes | 5 | 10.0, 9.8, 3.9, 3.5, 2.7 |
| 45 minutes | 1 | 3.5 |
| 60 minutes | 5 | 7.8, 5.5, 2.4 |

### b) ¹⁴C-GLUCOSE INCORPORATION INTO PIGEON BRAIN GLYCOCONJUGATES

Ninety microcuries of 1-¹⁴C glucose was injected intravenously into pigeons prior to a rest or training period, and the pigeons were sacrificed at intervals thereafter. The proteins of brain were then extracted and chromatographed from each pigeon brain and radioactivity determined on a scintillation counter. After exhaustive dialysis of the isolated brain protein groups, total radioactivity of the fraction was determined, then the fraction was subjected to stepwise acid hydrolysis with dialysis to free liberated sugars which were quantitatively determined. These sugar constituents were then separated by thin-layer chromatography, stained spots removed by vacuum suction and, together with blanks, counted. The results were as follows:

| Minutes after injection | Counts/minute total brain glycoconjugates | |
|---|---|---|
| | Training | Resting |
| 10 minutes | 2,000 | - |
| 20 minutes | 15,000 | - |
| 30 minutes | 21,000 | 1,000 |
| 45 minutes | 6,000 | 35,000 |
| 60 minutes | 1,500 | - |
| 120 minutes | - | 32,000 |
| 25.5 hours | - | 10,000 |

The time-course of incorporation is markedly different in the training state as compared to the resting state. In the training state, maximum incorporation occurs within 30 minutes at which time only one-twentieth as much is incorporated in the resting state.

### References

1.E. O. O'Callaghan,P. Sham, N. Takei, R.M. Murray. *Lancet* **337**, 1248-1250 (1991).
2. S. A. Mednick, R. A. Machon, M. O. Huttenen, D. Bonnet. *Arch Gen Psychiatry* **45,** 189-92 (1988.
3. E. O. O'Callaghan, P .C.Sham, N. Takei,G. Murray,G. Glover,E. Hare,R. M. Murray. *Schizophrenia Research* **9**, 138 (1993).
4. R. M. Murray, N. Takei, P. Sham, E. O. O'Callaghan, P. Wright. *Schizophrenia Research* **9**, 137 (1993). 5. P .C. Sham,C. J. MacLean, K. S.Kendler. *Schizophrenia Research* **9**, 139 (1993).
6. N . Takei,P. Sham,E. O. O'Callaghan,G.K. Murray, G. Glover, R. M. Murray.*Schizophrenia Research* **9**, 141 (1993).
7. D. Cotter,N. Takei, C. Larkin,P. Sham, R. M. Murray, M. Farrell, J . Oxford, E. O. O'Callaghan. *Schizophrenia Research* **9**, 147 (1993).
8. J. L Welham,J. J. McGrath,M. R. Pemberton.*Schizophrenia Research* **9**, 143 (1993).
9. R. E. Kendell, I. W. Kemp. *Arch Gen Psychiatry* **46**, 878-82, 1989.
10. M. Rahav,A. B. Goodman,M. Popper, S. P. Un. Am J Psychiatry **143**(10), 1249-1254 (1986); A. B. Goodman. *Schizophrenia Bulletin,* Accepted for publication.
11.F. E. Bloom.*Arch Gen Psychiatry* **50**, 224-227 (1993).
12. S. Akbarian,W. E. Bunney Jr, S. G. Potkin, S.B. Wigal,J. O. Hagman,C. A .Sandman,E. G. Jones. *Arch Gen. Psychiatry* **50**, 169-177 (1993).
13. S. Akbarian, A. Vinuela, J. J. Kim, S. G. Potkin, W. E. Bunney, E. G. Jones. *Arch Gen Psychiatry* **50**, 178-187 (1993).
14. J. A. Kovelman, A. B. Scheibel. *Biol Psychiatry* **19**, 1601-1621 (1984).
15. A. J. Conrad, A. B. Scheibel. *Schizophrenia Bulletin* **13(4)**, 577-587 (1987).
16. S. Bogoch. *Am J Psychiatry* **114**, 172, 1957.
17. *ibid. AMA Arch Neurol Psychiat* **80**, 221-224(1958).
18. *ibid. Nature* **184**, 1628-29(1959).
19. *ibid. J Biol Chem* **235**, 16-20 (1960).
20. *ibid. Am J Psychiatry* **116**, 743-747 (1960).
21. S. Bogoch, K.T. Dussik, P. G. Lever.*AMA Arch Gen Psychiat* **1**, 441-446 (1959).
22. S. Bogoch, K. T. Dussik, C. Fender P. Conran. *Am J Psychiatry* **117**, 409-415 (1960).
23. S. Bogoch,W. Sacks, G. Simpson. *Neurology* **13**.355 (1963).
24. S. Bogoch. *J Am Chem Soc* **79**, 3286 (1957).
25. *ibid. Biochem J* **68**, 319-324 (1958).
26. *ibid. Virology* **4**, 458-462 (1957).
27. *ibid.* in *Handbook of Neurochemistry,* Vol 1, A. Lajtha, Ed. (Plenum Press, N.1968).
28. *ibid,* in *Protein Metabolism of the Nervous System*, A. Lajtha, Ed.(Plenum Press, N.Y 1970) pp.555- 569.
29. S. Bogoch, P. Lynch, A. S. Levine. *Virology* **7**, 161-165 (1959).
30. S. Bogoch,E. S. Bogoch. *Nature* **183**, 53 (1959).
31. S. Bogoch, M. K. Paasonen, U. Trendelenburg. *Brit J Pharmacol* **18**, 325-329 (1962).
32. S. Bogoch, P. C. Rajam, P. C. Beival. *Nature* **204**, 73-75 (1964).
33. S. Bo goch, P. C. Belval, W. H. Sweet, W. Sacks, G. Korsh. Protides of Biol Fluids **XV**,129-135 (1968).
34. S. Bogoch. *The Biochemistry of Memory; with an Inquiry into the Function of the Brain Mucoids*. Oxford University Press, 1968.
35. *ibid,* in *The Nervous System, Vol 1, National Institutes of Neurological and Communicative Disorders and Stroke,* D.B. Tower,Ed.(Raven Press,N.Y.,1975) pp. 591-600.
36. S. Bogoch,K. T. Dussk,P. Conran. *New England Journal of Medicine* **264**, 251-258 (1961).
37. S. Bogoch,P. C. Belval,K. T. Dussik, P. Conran.*Am J Psychiairy* **119**, 128-133 (1962).
38. S. Bogoch, P. Evans. *Nature* **195**, 180 (1962).
39. R. J. Campbell, S. Bogoch, M. J. Scolaro, P. C. Belval. *Am J Psychiatry* **123**, 952-962 (1967).
40. E. Robins,A. B. Croninger, M. K .Smith, A. C. Moody. *Ann NY Acad Sci* **96**, 390-1(1962).
41. G. Christoni, R. Zappoli.*Am J Psychiatry* **117**, 246 (1960).
42. N. M. Papadopoulos, J. E. McLane, D. O'Doherty, W. C. Hess.*J Nerv Ment Dis* **128**, 450 (1959).
43. P. Sirota, H. Bessler, D. Allalouf,M. Daldetti,H. Levinsky.*Progress in Neuro-Psychopharmacology and Biological Psychiatry* **12 (1)**, 103-7 (1988).

EXAMPLE 1 Aglyco brain glycoprotein 108, and its specific antibody, are two aglyco products in brain and other malignancies. The antibody against aglyco 10B, produced in the body, causes cell death.

The evidence given in this Example, determinable in both the brain glycoconjugate 10B, and determinable by quantitation of the cytotoxic antibody made by the body against the resultant aglyco product of 10B (aglyco 10B'), illustrates the present invention. That the elevation in the absolute concentration of this antibody (anti-aglyco10B, or antimalignin) is observed in other common malignancies of different cell types, that is, cancer of the lung, breast, colon, etc. illustrates that aglyco pathology is a general pathological phenomenon, not just restricted to brain.

When glycoprotein 10B was identified as a normal 250 KD membrane constituent in human brain and shown to be involved in training in pigeon brain^{2.5-10}, one of the controls examined to test for the relationship of normal 10B to cell-cell interaction was brain malignancy. It was hypothesized that in brain glioblastoma, where apparent loss of contact inhibition of cell division was accompanied by unrestrained proliferation, the structure of 10B might be expected to be altered. Indeed, the structure of 10B was found to be markedly altered in brain glioblastoma: the carbohydrate groups were reduced in quantity by approximately 50% and there was a loss of heterogeneity. Where in the normal 10B there were 9 different carbohydrate constituents, in tumor 10B these were reduced to 5 or 6. Furthermore, the protein portion of 10B was overproduced or overexpressed 7 to 10 fold. Astrocytin was the name given to the 10KD peptide cleaved from brain tumor 10B. From glioblastoma cells grown in tissue culture, the equivalent to astrocytin, malignin, was isolated, and it proved to be a very close structural relative of astrocytin¹⁶. Malignin was so-named because in tissue culture the expresion of this peptide, and thus its concentration per mg. membrane protein extractable, increased with increased rate of cell division per unit time.

Recognin M was isolated from MCF7 malignant mammary cells. It is a 10 kD cancer polypeptide antigen rich in glutamic and aspartic acids, related to malignin isolated from glial brain tumors (Glu13,Asp9,His2)²¹. An IgM³⁷ auto-antibody against Recognin M, antimalignin, has been isolated from human serum²⁰, produced as mouse monoclonal^{31,33}, produced in human form by challenge of human lymphocytes with the antigen *in vitro* ^{35,37}, and has been isolated from malignant cells obtained at surgery and autopsy²⁴ by elution and immunoabsorption to its immobilized purified antigen.

### CLINICAL DATA

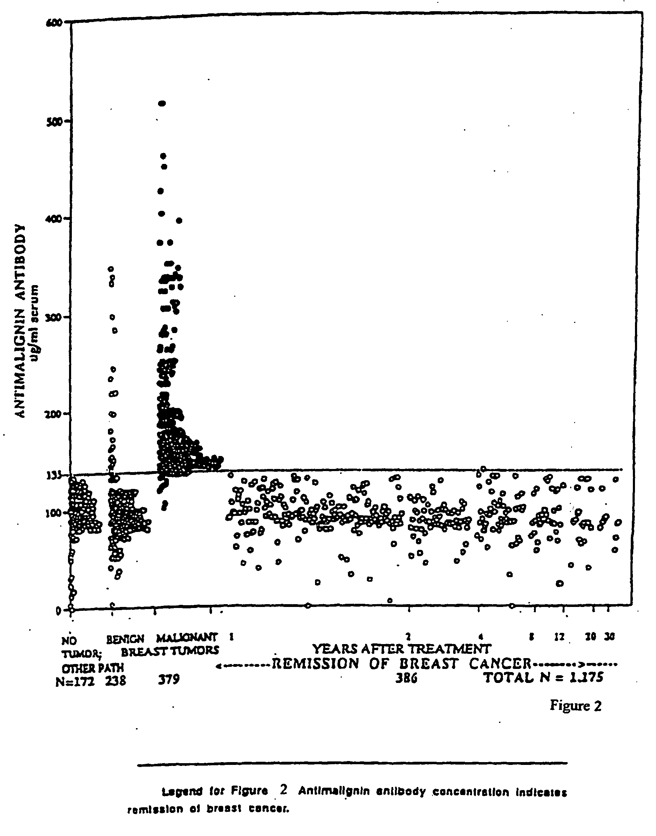

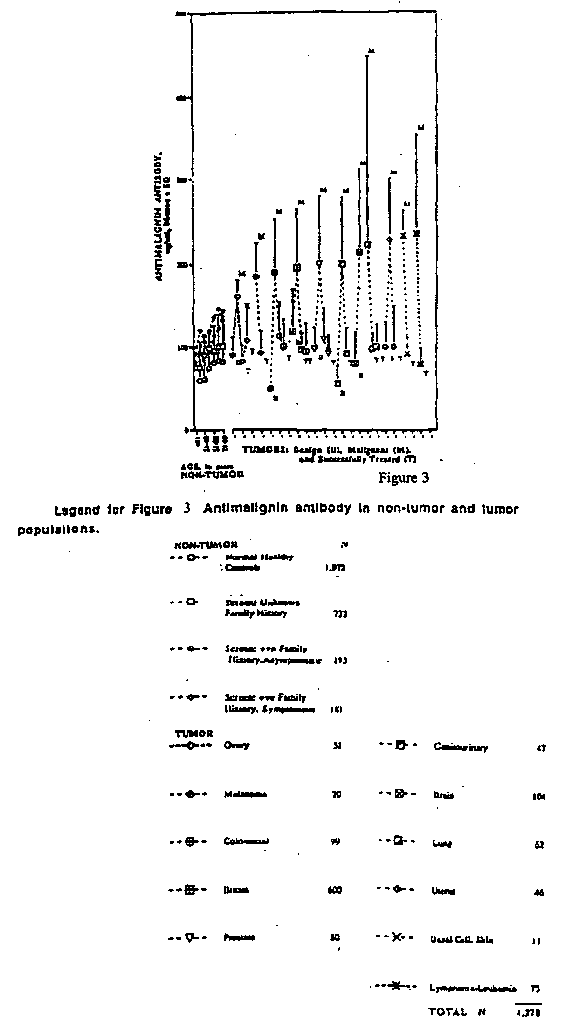

In a 20-year randomized mostly blind study involving several hundred physicians and three independent laboratories in the U.S., and three hospitals and one laboratory in the U.K. we have now found that the concentration of antimalignin in serum, in ug/ml, 1) of normal healthy non-tumor-bearing humans increases moderately each decade between the third and the seventh, as the risk of cancer increases (p<.001; N=1,972) (Figure 1), 2) increases earlier and more markedly in as yet apparently unaffected members of high-risk cancer families (p<.001; N=1,106) (Figure 1 and 3), and 3) is markedly increased in concentration in human serum within weeks of the occurence of malignant transformation to clinical breast cancer, but is not stigmatic (in the sense of invariant) since it returns to normal within 3 months of successful treatment^{39,41,44,45} and remains in the normal range even up to 27 years after successful treatment (Figures 2 and 3) (p<.001; false positives and false negatives <5% on first determination, <1% on repeat determination; N= 600).

An example of how antimalignin is not fixed but is modulated is shown in Table II. Readily biopsied cervix provided pathological evidence of transformation from dysplasia to the stage of frank invasive carcinoma; this was accompanied by marked elevation of the concentration of antimalignin. After surgical removal, antimalignin returned to normal within three months. Figures 2 and 3 show that these are statistically significant changes in both directions (p<.001).

Quantitative determination of serum antimatignin antibody is therefore of interest for use as a non-invasive biomarker to indicate successful results in breast cancer chemoprevention trials.

In addition, purified antimalignin antibody (MTAG), because of its demonstrated specificity in fluorescent and other chromogen staining of cell membranes in which the epitopes of malignin have become exposed^{16,27,28,30}, is applicable for use alone or as part of a battery of pre-dysplasia or dysplasia-based surrogate endpoint biomarkers in both individual and computerized cytometry.

### RELATION TO PATHOLOGICAL PROCESS: ANTIMALIGNIN IS AN INHIBITORY TRANSFORMATION ANTIBODY

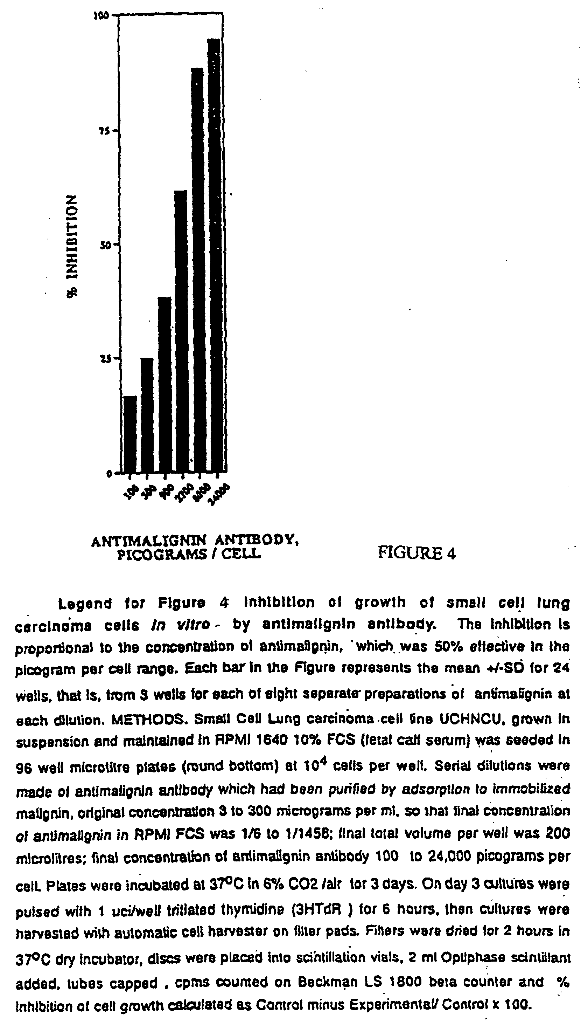

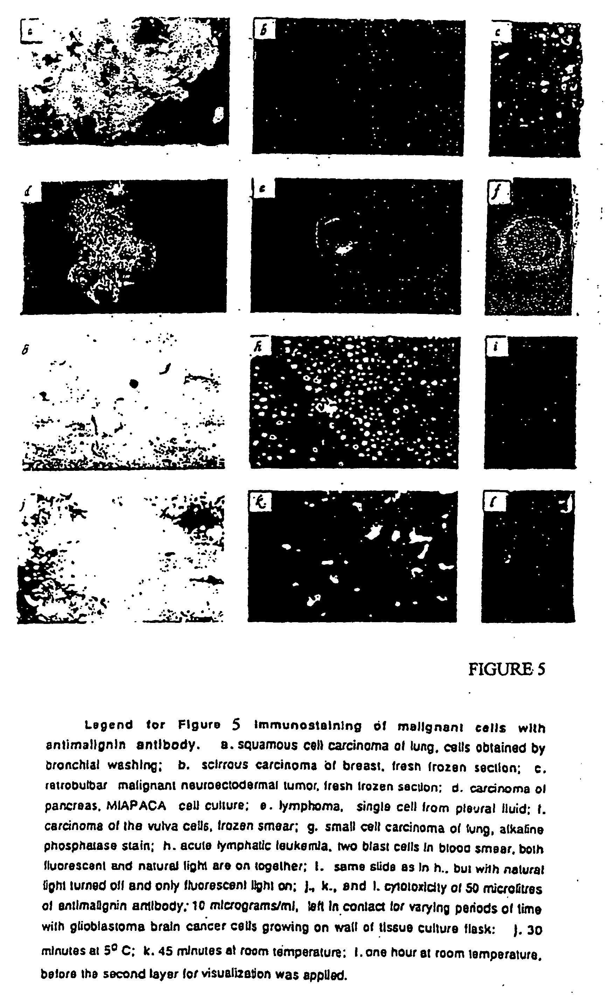

Cells which have undergone malignant transformation in humans may take years to, or may never, proliferate to become clinical cancer. If inhibition of proliferation is an immune process, as has been theorized^{1,3}, and suggested by the increased incidence of cancer in immunodeficiency disorders such as AIDS, there is no direct evidence inhuman cancer of such an immune process, and the responsible mechanisms are unknown. Antimalignin, in addition to the properties listed above, is quantitatively related to survival in patients^{32,34}. In vitro, antigen-purified human antimalignin binds to cancer cells regardless of cell type, is present in non-saturating amounts on cancer cells removed at surgery or autopsy, is inhibitory to the growth of small cell lung carcinoma cells at picograms (femtomoles as this is an IgM) of antibody per cell (Figure 4), and is cytotoxic to malignant glial cells (Figure 5 j,k and I).

Antimalignin antibody or derivates thereof, in human form, that contain its immunological specificity, is given subcutaneously or intravenously to animals or man, in amounts of one to ten grams per day and produces destruction of cancer cells.

Quantitative determination for antimalignin antibody: determined with immobilized malignin antigen (TARGET reagent, Brain Research, Inc., Boston). In the preparation of TARGET reagent, as previously described⁴⁴, human glioblastoma cells were grown in 250 ml sterile tissue culture flasks stacked in the horizontal position in a 37°C incubator until a monolayer of cells had covered the wall of the flask, freed from the wall with trypsin, scraped with spatula into a glass beaker, homogenized with a Branson sonifier, dialyzed, concentrated by perevaporation, centrifuged, chromatographed on a Cellex D column with stepwise elution with bufered solutions of decreasing pH, with the protein in each eluate quantified by adsorption at 280 mu. The last eluate, which contains malignin,eluted at its pK of approximately 2.7, was rechromatographed. The final preparation contained the malignin with the following composition: Glu 13 Asp9 Thr5 Ser5 Pro4 Gly6 Ala7 Val6 Met2 Ileu4 Leu8 Tyr3 Phe3 His2 Lys6 Arg51/2Cys1,and demonstrating a molecular weight of approximately 10K, and dimers and trimers thereof, on SDS gel and thin layer gel chromatography. Malignin was combined covalently with bromoacetylcellulose to produce immobilized TARGET reagent. To quantify the antimalignin antibody in cancer and norma isera, 0.2 ml samples of each serum specimen, in duplicate, were exposed to TARGET reagent with shaking for either two hours or 10 minutes, the bound antibody washed three times with cold NaCl, then released from the antigen by incubation with shaking with 0.25 molar acetic acid, centrifuged at 3000 rpm, the clear acetic acid supernatant read spectrophotometrically at O.D. 280, and the results converted to micrograms of protein. Results are given, as mean +/- S.D. for each serum, for NET TAG (=slow-binding antibody (2 hour contact of eluant with immobilized malignin) minus fast-binding antibody (10 minute contact of eluant with immobilized malignin), microgram/ ml serum⁴⁴.

### References:

1. Thomas L: Discussion paper P.B. Medawar, in *Cellular and Humoral Aspects of Hypersensitivity,* ed. H.S.Lawrence, Hober - Harper N.Y., 1959, p529
2. Bogoch *S:The Biochemistry of Memory:with an inquiry into the function of the brain mucoids* , Oxford University Press, 1968.
3. Bumet M: *Cellular Immunology* , Melbourne University Press, Cambridge University Press, 1969, pp252-28,.
4. Bogoch S: Inverse Relationship Between Cell Replication and Macromolecular Carbohydrate Concentration: A Distance Factor (DF) in Brain Tumors. Abstr. *3rd Intnl meeting of the Intnl Soc. for Neurochemistry,* July, 1971.
5. Bogoch S: Brain Glycoprotein 10B: Further Evidence of the "Sign-Post"Role of Brain Glycoproteins in Cell Recognition, its Change in Brain Tumor, and the Presence of a "Distance Factor". Chap P. Mandel, and I.G. Morgan. Plenum Press, 1972, pp. 39-52.
6. Bogoch S: Brain Glycoproteins and Inter-Cell Recognition: Tay-Sachs' Disease and Intemeuronal Recognition. Chapter In *Sphingolipids, Sphingolipidoses and Allied Disorders*, Eds. B.W. Volk and S.M. Aronson.Plenum Press, 1972, pp. 127-149.
7. Bogoch S: Astrocytin: Purified Immunologically Active Component of Brain Glycoprotein 10B. Abstracts of 4th Intnl. Mtg., Intnl. Soc. for Neurochemistry, Tokyo, 1973.
8. Bogoch S: Brain Glycoproteins and Learning: New Studies Supporting the "Sign-Post" Theory. *Abstracts of 4th Intnl Mtg. Intnl. Soc. for Neurochemistry*, Tokyo, 1973.
9. Bogoch S: Brain Glycoproteins and Leaming: New Studies supporting the "Sign-Post" Theory. Ch. *Current Biochemical Approaches to Leaming and Memory.* Eds. W.B. Essman and S. Nakajima. Spectrum Publications, 1973.
10. Bogoch S: Glycoproteins and Brain Circuitry: the "Sign-Post" Theory in Normal Memory Function, and the Regressive States of Brain Tumors and in the Psychoses. Chapter in *Biological Diagnosis of Brain Disorders*. Ed. S. Bogoch. Spectrum-Halstead-Wiley, New York, 1974.
11. Bogoch S: The Detection of malignant Gliomas in Brain by the QuantitativeProduction In Vitro of TAG (Target-Attaching-Globulins) from Human Serum. Chapter in *Biological Diagnosis of Brain Disorders.* Ed. S. Bogoch. Spectrum-Halstead-Wiley, New York, 1974.
12. Bogoch S: The "Sign-Post" Function of Brain Glycoproteins: Order and Disorder. In *The Nervous System. 25th Anniversary Volume, National Institutes of Neurological and Communicative Disorders and Stroke:Vol. 1, The BasicNeurosciences*. Ed. D.B. Tower. Raven Press, 1975, pp 591-600.
13. Bogoch S: Malignin: A Polypeptide Related to Brain Malignancy. *Transactions of the American Society for Neurochemistry*, 7 (1),239, 1976.
14. Bogoch S: Brain Glycoproteins and Recognition Functions: Recognins and Cancer. In *Current Trends in Sphingolipidoses and Allied Disorders.* Eds. BL Volk and L Schneck. Plenum Press, New York, 1976. pp. 555-566.
15. Komblith PL, Caswell PA, BogochS,Callahan LV, and Dreyfus J: Effects of Diphenylhydantoin on Cultured Human Glial Cells. *In Vitro:,April* 1976, p324.
16. Bogoch S: Astrocytin and Malignin: Two Polypeptide Fragments (Recognins) Related to Brain Tumor. *Natnl. Cancer Institute Monograph* 46:133-137, 1977.
17. Bogoch S: Malignin: Cancer Polypeptide. In *Mechanisms, Regulation and Special Functions of Protein Synthesis in the Brain.* Eds. S Roberts, A Lajtha and WH Gispen. Elsevier Press, Amsterdam and New York, 1977, pp 433-440.
18. Bogoch S: Malgnin: Polypeptide Cancer Recognin. *Abstracts of the International Society for Neurochemistry* Annual Meeting, Copenhagen, 1977.
19. Bogoch S: Recognins and their Chemoreciprocats. In *Behavioral Neurochemistry*. Eds. J.M.R. Delgado and F.V. DeFeudis. Sectrum-Wiley Press, New York, 1977, pp 197-222.
20. Harris JH, Gohara A and Bogoch S: New Immunodiagnostic Techniques for CNS Tumors. *J. Neuropath. Exper. Neurol*. **37**:623, 1978.
21. Bogoch S and Bogoch E: New Cancer Recognins, L and M, Immunochemically Related to Brain Malignin. *Transactions of the American ociety for Neurochemistry* 10(1):78, 1979.
22. Bogoch S, Bogoch E, Fager CA, Goldensohn ES, Harris JH, Hickok DF, Lowden JA, Lux WE, Ransohoff J and Walker MD: Elevated Serum Anti-Malignin Antibody in Glioma and Other Cancer Patients: A Seven-Hospital Study. *Neurology* 29: 584, 1979.
23. Bogoch S and Bogoch ES: Disarmed Anti-Malignin Antibody in Human Cancer. *The Lancet* 1:987, 1979.
24. Bogoch S and Bogoch ES: Production of Two Recognins Related to Malignin: Recognin M from Mammary MCF-7 Carcinoma Cells and Recognin L from Lymphoma P3J Cells. *Neurochemical Research* 4: 467-473, 1979.
25. Bogoch S: Cancer Diagnostic Reagents.British patent 1532803, 1979.
26. Bogoch S and Bogoch ES: Tumor Markers: Malignin and Related Recognins Associated with Malignancy Rather than with Cell Type. In *Neurochemistry and Clinical Neurology,* Eds. L Battistin, G Hashim, A Lajtha, Alan R. Liss, Inc. New York 1980, pp 407-424.
27. Redmond FA: Antimalignin Antibody. Doctoral Thesis, Medical College of Ohio at Toledo, 1980.
28. Harris JH, Gohara A, Redmond F, Bogoch S and Bogoch ES: Immunofluorescent and SerologicStudies with Anti-Malignin Antibody. Chapter in *Perspectives of Immunology* .Ed. SA Rosenberg, Academic Press, New York, 1980, pp 571-582.
29. Bogoch S and Bogoch ES: Quantitative determination of Anti-Malignin Antibody. Chapter in *Perspectives in Immunology* .Ed. S.A. Rosenberg. Academic Press, New York, 1980, pp 693- 696.
30. Redmond FA, Harris JH, Loeb TL, Bogoch S, Bogoch ES and Gohara A: Studies of Human Tumors with Anti-Malignin Antibody (MTAG). *FASEB Ann.Mtg. Abstract* no. 40139, 1980.
31. Bogoch S, Bogoch ES and Tsung Y-K: Monoclonal Anti-Malignin Antibodies. *The Lancet*1:141-142, 1981.
32. Bogoch S, Bogoch E, Fager CA, Harris JH, Ambrus JL, Lux WE and Ransohoff JA: Detemination of Anti-Malignin Antibody and Malignin in 1,026 Cancer patients and Controls: Relation of Antibody to Survival. *J. Medicine* 13: 22-42, 1982.
33. Bogoch S and Bogoch ES: Malignin, Anti-Malignin Antibody and *Scantag.Protides of Biol.* *Fluids*, Pergamon, N.Y. 29: 337-352, 1983.
34. Bogoch S , Bogoch ES, Antich P, Dungan SM, Harris JH, Ambrus JL and Powers N: Elevated Levels of Anti-Malignin Antibody are Quantitatively Related to Longer Survival in Cancer Patients. *Protides of Biol. Fluids*, Pergamon, N.Y. 31: 739-747, 1984.
35. Bogoch S, Bogoch ES and Iliescu VM: In Vitro Production of the General Transformation Antibody Related to Survival in Human Cancer Patients: Anti-Malignin Antibody(AMA). *Cancer Detection and Prevention* 8:033, 1985.
36. Bogoch S and Bogoch ES: Increased Accuracy of Anti-Malignin Antibody Determination in Unstored Sera Permits Screening. *Cancer Detection and Prevention* 11:100, 1987.
37. Bogoch S, Bogoch ES and Iliescu VM: In Vitro Production of the general Transformation Antibody Related to Survival in Human Cancer Patients:Antimalignin Antibody. *Cancer Detection and Prevention* 12:313-320, 1988.
38. Thomthwaite JT, DerhagopianR and ReimerW:Antimalignin Antibody Determination in Patients with Suspicious Mammograms.*Proc Annu Meet Am Assoc Cancer Res* 31, A1550,1990.
39. Bogoch S and Bogoch ES: Malignin antibody and early malignancy. *The Lancet* 337:977,1991.
40. Boone CW, Kelloff GJ and Steele VE: Natural History of intraepithelial Neoplasia in Humans with Implications for Cancer Chemoprevention Strategy. *Cancer Research* 52:1651-1659. 1992.
41. Bogoch S and Bogoch ES: Malignin antibody Returns to Normal Alter Successful Treatment of Breast Cancer. *Cancer Detection and Prevention*17(1): 180, 1993.
42. Bogoch ES and Bogoch S: Malignin Antibody As Early Warning. *Cancer Detection and Prevention* 17(1): 229, 1993.
43. Boone CW and Kelloff GJ: Intraepithelial Neoplasia Surrogate Endpoint Biomarkers and Cancer Chemoprevention. *J. Cell Biochem* 17F(Suppl): 37-48, 1993.
44. Abrams MB, Bednarek K.,Bogoch S. Bogoch ES,Dardik HJ, Dowden R, Fox SC, Goins EE, Goodfried G. Herrman RA, Imperio J, Jackson W, *Keuer* S, Killackey M, Kimel G. Layton RE. Liebentritt AH, Marsden,McCabe JL, Menasha M,OrtenK,PasmantierM,PillaiT,Pillai VB. Probst W . Reimer W. Smith S. Thomthwaite J, Turner WJ and Whitlock RT: Early Detection and Monitoring of Cancer in Routine Practice with the Antimalignin Antibody Test. *Cancer Detection and Prevention*: 1994;18(1): 65-78.
45. Thomthwaite JT, Derhagopian R and ReimerW: Determination of Antimalignin in Patients with Suspicious Mammograms. Cancer, Submitted for Publication 1993.

### EXAMPLE 2 The carbohydrate constituents of brain glycoconjugates, reduced in concentration in the presence of brain tumors, can be increased towards normal concentration by certain drugs, eg. Diphenylhydantoin (DPH).

Mice were divided into two groups, one-half which were inoculated with subcutaneous brain ependymomas, and one half which were not. Each of these two groups was divided into two groups: one-half would receive daily subcutaneous DPH, 1 mg/kg body weight, and one-half which received no DPH but saline injections only. Animals were sacrificed when the ependymoma grew sufficiently just to break the skin surface. There was a significant reduction in the growth of tumors when DPH was given. The brains were pooled for each subgroup, and extracted for protein-bound hexose as in the Preparative Example, with the following results:

| Brain Protein-Bound Hexose, as % of Protein | | |
|---|---|---|
| Tumor | Without DPH | With DPH |
| Absent | 4.6, 5.5 | 7.9, 8.0 |
| Present | 2.6, 2.3 | 6.8, 7.0 |

It should be noted that DPH produces an increase in the concentration of brain protein-bound hexose in the absence of tumors as well as in their presence.

### EXAMPLE 3 Structure of Aglyco10B

A. lodobenzoic Acid Hydrolysis of Aglyco10B Followed by Edman Degradation Yielded the Peptide ("lodopeptide") with the following sequence:

B. Mass spectrometry followed by calculations using MacBioSpec Software Manual 013048-A, PESCIEX, Perkin Elmer Sciex Instruments, yielded the following properties of the peptide SEQ ID NO:1 :
N-Terminal Group: Hydrogen C-Terminal Group: Free acid
MH+ Monoisotopic Mass = 1847.9656 amu HPLC index = 43.40
MH+ Average Mass = 1849.0998 amu Bull & Breese value = -80
Isoelectric Point (pl) = 8.0 Elemental Composition: C₈₄H₁₃₁ N₂₂ O₂₅

C. Mass spectrometry:The sequence of SEQ ID NO:1 in 9A. above obtained by lodobenzoic acid hydrolysis was independently confirmed by mass spectrometry of fragments of Aglyco10B obtained by four different acid hydrolyses of Aglyco10B in solution and of Aglyco10B immobilized on bromoacetyl-cellulose (Aglyco10BC; or A10BC) as below. These hydrolyses produced 13 overlapping hydrolytic fragments (two of which, 6-10 and 6-12 below, were each obtained by two different hydrolytic methods). Taken together, these overlapping fragments independently confirmed the peptide sequence of SEQ ID NO:1 as follows:

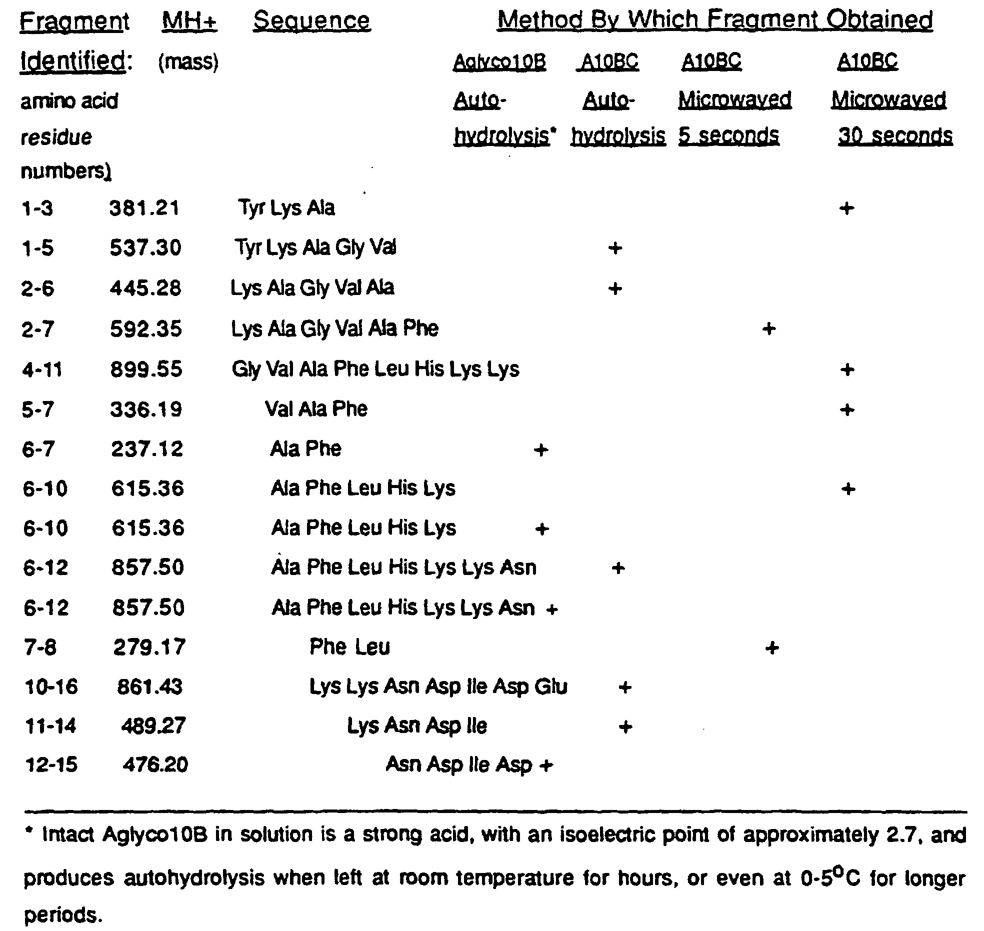

C. Trypsin Hydrolysis of Aglyco10B Followed by Edman Degradation Yielded the peptide SEQ ID NO:2 with the following sequence:

D. Mass spectrometry of hydrolytic fragments of Aglyco10B followed by calculations using MacBioSpec Software Manual 013048-A, PESCIEX, Perkin Elmer Sciex Instruments, yielded the following properties of the peptide SEQ ID NO:2 Gly Leu Ser Asp Gly Ser Asn Thr Glu Ser Asp lie
N-Terminal Group: Hydrogen C-Terminal Group: Free acid
MH+ Monoisotopic Mass = 1194.5126 amu HPLC index = 0.70
MH+ Average Mass= 1195.1817 amu Bull & Breese value = 2690
Isoelectric Point (pl) = 4.4 Elemental Composition: C₄₆ H₇₆ N₁₃ O₂₄

E. Mass spectrometry:The sequence of amino acids 2-11 of SEQ ID NO:2 in 2C. above obtained by trypsin hydrolysis was independently confirmed by mass spectrometry of fragments of Aglyco10B obtained by four different acid hydrolyses of Aglyco10B in solution and of Aglyco10B immobilized on bromoacetyl-cellulose (Aglyco 10B-cellulose; or A10BC) as below. These hydrolyses produced 7 overlapping hydrolytic fragments which independently confirmed 2-11 of the peptide sequence of SEQ ID NO:2 Gly Leu Ser Asp Gly Ser Asn Thr Glu Ser Asp Ile as follows:

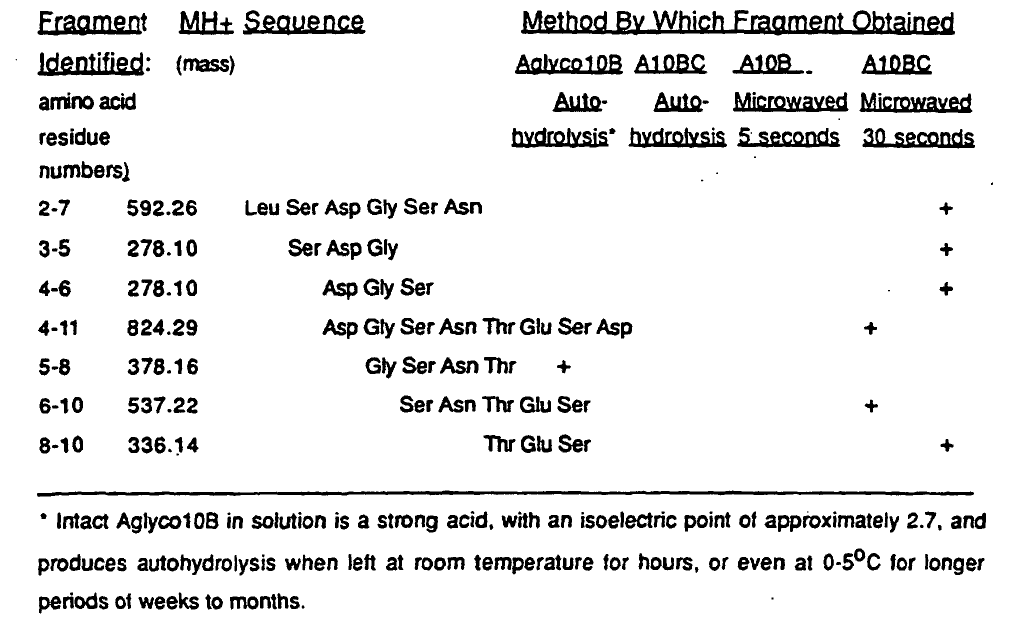

F. CNBr hydrolysis of Aglyco10B Followed by Edman Degradation Yielded the Dipeptide SEQ ID NO:3 with the following sequence: Met Asp

G. USE OF AGLYCO10B, A10BC, INTACT 10B, SEQ ID NO:1 OR SEQ ID NO 2 AS A VACCINE. Approximately 1mg of any of the above glycoprotein or protein fragments, injected twice or three times subcutaneously in animals including man, produce antimalignin antibody and cellular response for the prevention or treatment of cancer.

### SEQUENCE LISTING:

(1) GENERAL INFORMATION PATENT APPLICATION
   (i) APPLICANT: SAMUEL BOGOCH
      ELENORE S. BOGOCH
   (ii) TITLE OF INVENTION: AGLYCO PRODUCTS
   (iii) NUMBER OF SEQUENCES: 2
   (iv) CORRESPONDENCE ADDRESS:
      (A) ADDRESSEE: SAMUEL BOGOCH
      (B) STREET: 46 EAST 91 ST STREET
      (C) CITY: NEW YORK
      (D) STATE: NEW YORK
      (E) COUNTRY: U.S.A.
      (F) ZIP: 10028
   (v) COMPUTER READABLE FORM:
      (A) MEDIUM TYPE: FLOPPY DISK
      (B) COMPUTER: MAC
      (C) OPERATING SYSTEM: MAC CLARIS XTND
      (D) SOFTWARE: MACWRITE
   (vi) CURRENT APPLICATION DATA:
      (A) APPLICATION NUMBER:
      (B) FILING DATE:
      (C) CLASSIFICATION:
   (vii) PRIOR APPLICATION DATA: N/A
   (viii) ATTORNEY/AGENT INFORMATION: N/A
   (ix) TELECOMMUNICATION INFORMATION:
      (A) TELEPHONE: 212-831-3070
      (B) TELEFAX: 212-831-6645
      (C) TELEX: -
(2) INFORMATION FOR SEQUENCE ID NO 1:
   (i) SEQUENCE CHARACTERISTICS:
      (A) LENGTH: 16
      (B) TYPE: AMINO ACIDS
      (C) STRANDEDNESS: -
      (D) TOPOLOGY: UNKNOWN
   (ii) MOLECULE TYPE
      (A) DESCRIPTION: PEPTIDE
   (iii) HYPOTHETICAL: -
   (iv) ANTI-SENSE: -
   (v) FRAGMENT TYPE: -
   (vi) ORIGINAL SOURCE:
      (A) ORGANISM: HUMAN BRAIN GLIOMA, SUBCULTURE
      (B) STRAIN: N/A
      (C) INDIVIDUAL ISOLATE: N/A
      (D) DEVELOPMENTAL STAGE: UNKNOWN
      (E) HAPLOTYPE: -
      (F) TISSUE TYPE: HUMAN BRAIN GLIOMA, SUBCULTURE
      (G) CELL TYPE: GLIOMA
      (H) CELL LINE: GUOMA
      (I) ORGANELLE: N/A
   (vii) IMMEDIATE SOURCE:
      1) AGLYCO10B, ISOLATED FROM GLIOMA;
      2) AGLYCO10B, IMMOBILIZED ON BROMOACETYLCELLULOSE.

      (A) LIBRARY: N/A
      (B) CLONE: N/A
   (viii) N/A
   (ix) N/A
   (x) N/A
   (xi) SEQUENCE DESCRIPTION: SEO ID NO:1:
   Tyr Lys Ala Gly Val Ala Phe Leu His Lys Lys Asn Asp Ile Asp Glu
   1               5                   10                  15
(3) INFORMATION FOR SEQ ID NO 2:
   (i) SEQUENCE CHARACTERISTICS:
      (A) LENGTH: 12
      (B) TYPE: AMINO ACIDS
      (C) STRANDEDNESS:
      (D) TOPOLOGY: UNKNOWN
   (ii) MOLECULE TYPE
      (A) DESCRIPTION: PEPTIDE
   (iii) HYPOTHETICAL: -
   (iv) ANTI-SENSE: -
   (v) FRAGMENT TYPE: -
   (vi) ORIGINAL SOURCE:
      (A) ORGANISM: HUMAN BRAIN GLIOMA, SUBCULTURE
      (B) STRAIN: N/A
      (C) INDIVIDUAL ISOLATE: N/A
      (D) DEVELOPMENTAL STAGE: UNKNOWN
      (E) HAPLOTYPE: -
      (F) TISSUE TYPE: HUMAN BRAIN GLIOMA, SUBCULTURE
      (G) CELL TYPE: GLIOMA
      (H) CELL LINE: GLIOMA
      (I) ORGANELLE: N/A
   (vii) IMMEDIATE SOURCE:
      1) AGLYCO10B, ISOLATED FROM GUOMA;
      2) AGLYCO10B, IMMOBILIZED ON BROMOACETYLCELLULOSE.

      (A) LIBRARY: N/A
      (B) CLONE: N/A
   (viii) N/A
   (ix) N/A (x) N/A
   (xi) SEQUENCE DESCRIPTION: SEQ ID NO: 2:

G. A chromophore group ("Chromophore") is present in Aglyco10B which results in Aglyco10B being slightly yellow in a concentrated solution (>100 ug/ml). The Chromophore remains with Aglyco10B throughout all phases of its purification including high pressure liquid chromatography.

When oxygen is replaced by nitrogen by evacuating and heat-sealing the glass tube which contains the Aglyco10B solution, the Aglyco10B solution turns dark green.
When the glass tube is opened to the air, the solution immediately returns to the yellow color.

Since this transition from yellow in the oxygenated state to dark green in the anoxic state and back to yellow in the oxygented state is a reversible transition seen only in relation to the presence or absence of oxygen, it is concluded that the color change is the property of reversible oxidation:reduction of the Chromophore.

H. Cloning the gene for Aglyco10B. By methods well known in the art, the gene for Aglyco10B and thus for 10B as well, with full amino acid sequence, is derived using either the peptide SEQ ID NO:1 of Example 9A above or the peptide SEQ ID NO:2 of Example 9C above , or both, to construct RNA, and a cDNA probe of normal and transformed glial cell libraries. The gene for Aglyco10B and of 10B permits their use in their entirety, or with fragments of the structure, as vaccines. In addition, the entire gene or parts thereof can be introduced into various well known expression systems to produce intact 10B glycoconjugated protein or Aglyco10B, and these products used as vaccines in the patients, or in animals to produce the specific antibodies to the intact molecules or fragments thereof. Since 10B is known to be involved in recognition and cognitive training in the whole animal, it can be administered to improve cognitive function in animals and man (see Alzheimer's Disease below).

### 10. Aglyco products in Alzheimer's Disease.

Alzheimer's Disease is a devastating wasting disease in which nerve cell loss is manifested by cognitive disturbances, predominately memory loss in early phases. Certain derivatives of the Amyloid Precursor Protein (APP) are responsible for the abnormal brain deposits characteristic of this disease. Although APP is known to be a glycoprotein, no attention as yet has been paid to the carbohydrates in APP. Because of this invention, aglyco products can be detected 1) directly by isolation and characterization of the derivatives of APP in cerebrospinal fluid, which have decreased carbohydrate components; and 2) indirectly, by detection and quantification in patient's serum of antibodies to Aglyco APP.

Early pre-amyloid deposits in brain have been visualized before the neurofibrillary tangles, the products of nerve cell destruction, have appeared and joined the 'mature' Alzheimer plaque. These pre-amyloid deposits, like the mature plaques, contain Ab peptides, 39-42 amino acid residues long degradation products of APP. While the transition from pre-amyloid to mature amyloid plaques is likely, since pre-amyloid deposits are present years before degenerating neurites appear in Down's syndrome patients' brain. Pre-amyloid deposits can be more amenable to treatment, that is to dissolution and excretion, before damage to nerve cells occurs. Protection against further accumulation of plaques can be afforded by administering diphenylhydantoin (DPH) which this invention demonstrates increases the concentration of protein-bound hexose in brain (see Example 8 above).

Examples of other conditions in which new aglyco antigens may be exposed and give rise to aglyco antibodies are in Parkinson's Disease and multiple sclerosis, and this invention should be applicable to these and other disorders, both of brain and other organs, both with regard to diagnosis and treatment as above.

## Claims

1. A peptide where the sequence of the amino acids is tryosine-lysine-alanine-glycine-valine-alanine-phenylalanine-leucine-histidine-lysine-lysine-asparagine-aspartic acid-isoleucine-asparagine-glutamic acid or derivative thereof.

2. A peptide where the sequence of the amino acids is glycine-leucine-serine-aspartic acid-glycine-serine-asparagine-theronine-glutamic acid-serine-aspartic acid-isoleucine or derivative thereof.

3. Use of a peptide as defined in claim 1 or 2 for the preparation of a medicament for the treatment of cancer.

4. An ex vivo process for diagnosing cancer or susceptibility to cancer in a subject related to expression or activity of a polypeptide according to claim 1 or 2 in a subject comprising analysing for the presence or amount of said polypeptide or an antibody to said polypeptide, in a sample derived from said subject.

5. A vaccine composition comprising a polypeptide as defined in claim 1 or 2 and a pharmaceutically effective carrier.

## Patentansprüche

1. Peptid, bei dem die Sequenz der Aminosäuren Tyrosin-Lysin-Alanin-Glycin-Valin-Alanin-Phenylalanin-Leucin-Histidin-Lysin-Lysin-Asparagin-Asparaginsäure-Isoleucin-Asparagin-Glutaminsäure oder ein Derivat davon ist.

2. Peptid, bei dem die Sequenz der Aminosäuren Glycin-Leucin-Serin-Asparaginsäure-Glycin-Serin-Asparagin-Threonin-Glutaminsäure-Serin-Asparaginsäure-Isoleucin oder ein Derivat davon ist.

3. Verwendung eines Peptids, wie es in Anspruch 1 oder 2 definiert ist, für die Herstellung eines Medikaments für die Behandlung von Krebs.

4. Ex vivo-Verfahren zum Diagnostizieren von Krebs oder von Empfänglichkeit für Krebs in einem Subjekt, welches mit Expression oder Aktivität eines Polypeptids gemäß Anspruch 1 oder 2 in einem Subjekt in Zusammenhang steht und welches umfaßt, daß man eine Analyse hinsichtlich des Vorhandenseins oder der Menge des Polypeptids oder eines Antikörpers gegen das Polypeptid in einer Probe, die von dem Subjekt erhalten wurde, durchführt.

5. Impfstoffzusammensetzung, die ein Polypeptid, wie es in Anspruch 1 oder 2 definiert ist, und einen pharmazeutisch wirksamen Träger umfaßt.

## Revendications

1. Peptide dont la séquence d'aminoacides est tyrosine-lysine-alanine-glycine-valine-alanine-phénylalanine-leucine-histidine-lysine-lysine-asparagine-acide aspartique-isoleucine-asparagine-acide glutamique, ou un de ses dérivés.

2. Peptide dont la séquence d'aminoacides est glycine-leucine-sérine-acide aspartique-glycine-sérine-asparagine-théronine-acide glutamique-sérine-acide aspartique-isoleucine, ou un de ses dérivés.

3. Utilisation d'un peptide tel que défini dans la revendication 1 ou 2 pour la préparation d'un médicament destiné au traitement du cancer.

4. Procédé *ex vivo* pour le diagnostic du cancer ou de la sensibilité au cancer chez un sujet en rapport avec l'expression ou l'activité d'un polypeptide suivant la revendication 1 ou 2 chez un sujet, comprenant une analyse pour déterminer la présence ou la quantité dudit polypeptide ou d'un anticorps contre ledit polypeptide dans un échantillon provenant dudit sujet.

5. Composition de vaccin comprenant un polypeptide tel que défini dans la revendication 1 ou 2 et un support pharmaceutiquement efficace.
